# EUROPEAN PATENT APPLICATION

(11) **EP 3 663 894 A2**
(43) Date of publication of application: **10.06.2020**
(21) Application number: 18838413.5
(22) Date of filing: 26.07.2018
(51) Int. Cl.: G06F 3/01

(54) **DEVICE AND METHOD FOR SIMULATING AND TRANSMITTNG CONTACT EXTEROCEPTIVE SENSATIONS**

(30) Priority: 26.07.2017 RU 2017126891
(71) Applicant: Belomoev, Fedor Valentinovich, St.Petersburg 196605 (RU)
(72) Inventor: Belomoev, Fedor Valentinovich, St.Petersburg 196605 (RU)
(74) Representative: Tobiasz-Dumania, Katarzyna
(86) International application number: PCT/RU2018/000499
(87) International publication number: WO 2019/022644

(57) **Abstract**

The present invention relates to information technologies, and is designed as a glove for simulating tactile exteroceptive sensations occurring as a result of stimulation of receptors located on the inner and outer sides of a palm and on fingers. A device is manufactured, for example, from a textile material having stimulators arranged thereon, which directly influence receptors through a skin surface and simulate tactile sensations.

## Description

### Field of invention

The present invention relates to information technologies and can be used for simulating tactile exteroceptive sensations occurring as a result of stimulation of receptors located on skin surface using computers, mobile phones and other electronic devices.

In particular, the present invention provide a device in form of a glove having vibrating, touching, locomotory, heating and cooling elements used to stimulate receptors for simulating tactile exteroceptive sensations.

### Background of the disclosure

Currently, the use a series of game controllers and gamepads is known, the game controllers and gamepads are two-handed remote controllers with buttons and symmetrically placed analogues joysticks for the thumbs with vibration function as a feedback means. However, such devices transmit only general vibration to the user's hands at episodic moments.

US patent No. 6,677,932 describes a system and method for recognizing touch typing and creating tactile feedback with a user. This device provides tactile feedback to simulate pressing a mechanical button. The goal of the invention is to provide tactile feedback when typing on the keyboard in order to prevent an erroneous hit of the finger past the button In this process only touching the button is simulated, and only an audio or vibration signal is released.

A system and method for applying vibration to a human body is described in US patent No. 8,139,803. This vibration system includes a vibrator capable of converting an electrical signal into vibration. In one case, the vibrator is located on, or near the human body on the pectoral muscle and at a distance from the sternum. In another embodiment, the vibrator is located on or near the human body in order to generate vibrations on the surface of the body. The described device does not simulate the transmission of tactile sensations, since it transmits only vibration sensations to or near the chest area.

There are many variants for gloves of virtual reality, which contain sensors for the location and movement of the fingers to control the "virtual hand" in virtual reality programs. Some of the gloves contain vibro-drive systems that provide feedback and simulation of tactile sensations. The examples of such gloves are Dexmo glove of Dextra Robotics company (http://www.dextarobotics.com), Peregrine glove (http://theperegrine.com) and Manus VR glove (https://manus-vr.com).

However, the known gloves do not provide the entirety of tactile sensations, including sensations of vibration, touch, movement, texture and temperature of objects in virtual reality.

### Summary of the disclosure

The object of the present invention is to provide a compact, easy-to-manufacture and use device for simulating various tactile sensations, as well as a method for simulating various tactile sensations using the device.

This goal was achieved by creating a device in the form of a glove, which uses tactile actuation on receptive fields to simulate tactile sensations. The proposed device is simple and convenient to use, and allows the perception of simulated reality in a portable and mobile mode.

The device of the present invention comprises:
- a glove made of elastic material,
- stimulants of mechanical, dynamic and thermal effects, fixed at the glove areas, contacting with the fingers and palm of the hand at the areas of location of Meissner's, Pacinian, Ruffini's and Krause's mechanoreceptors, and Merkel nerve endings,
- control and communication unit connected to these stimulants and communicating with control software of a computer or a smartphone,
- power supply.

One of the embodiments of the present invention is a device in the form of a glove for simulating contact exteroceptive sensations on the skin, in this case on the fingers and palm of the hand, by means of stimulants located in it, which are synchronized with each other and transmit mechanical, dynamic and thermal actuations, namely actuations of vibration, compression, expansion, displacement, pressure, heating and cooling.

In one embodiment, the present invention can be used as an addition to visualizing an object, event, or transmitted sensations to create the most perceptual image.

In one embodiment, the device of the present invention comprises tactile stimulators used to form tactile sensations located on the tips of the fingers and on the palm of the hand.

In one embodiment, the device of the present invention contains vibratory stimulants used to simulate sensations of vibration located on the inner side of the phalanges of the fingers, as well as on the inner side of the hand.

In one embodiment, the device of the present invention comprises thermal stimulants used to simulate temperature sensations located on the inner side and on the outer side of the hand.

In one embodiment, the device of the present invention comprises a fixing member on the arm wrist.

In one embodiment of the method according to the present invention, imitation and reception of contact exteroceptive sensations is carried out by the device described above. The transmitted sensations are formed by sequential or simultaneous actuations on the receptors of the fingers and palm of the hand performed by stimulators controlled by software installed on a computer or smartphone connected to the device by wire or wirelessly, through the control and communication unit of the device.

### Brief Description of the Drawing

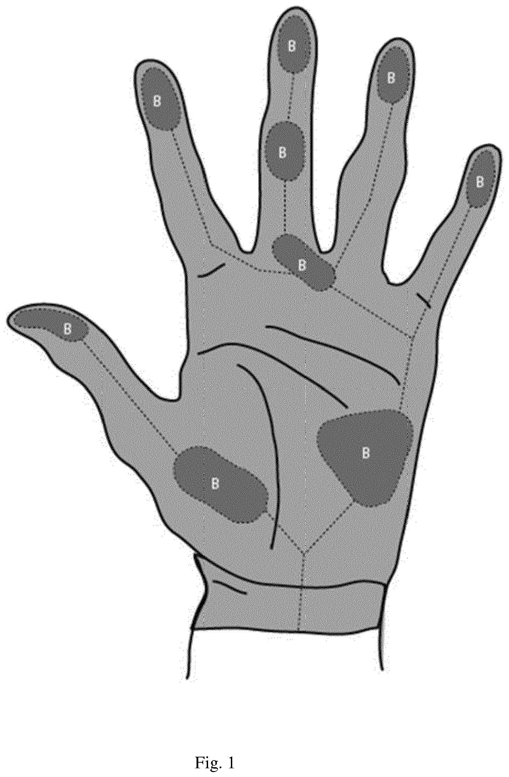

Fig. 1 shows one embodiment of the device according to the present invention in the form of a glove, where the letter "B" on the inner side of the palm of the glove marks areas of receptive actuation.

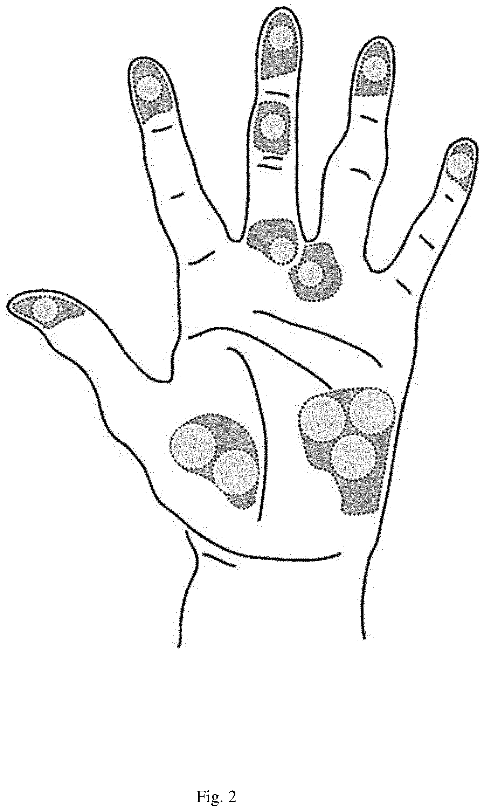

The most effective zones of receptive actuation on the receptive field on the inner side of the palm containing a maximum accumulation of all stimulated tactile mechanoreceptors are noted on Figure 2.

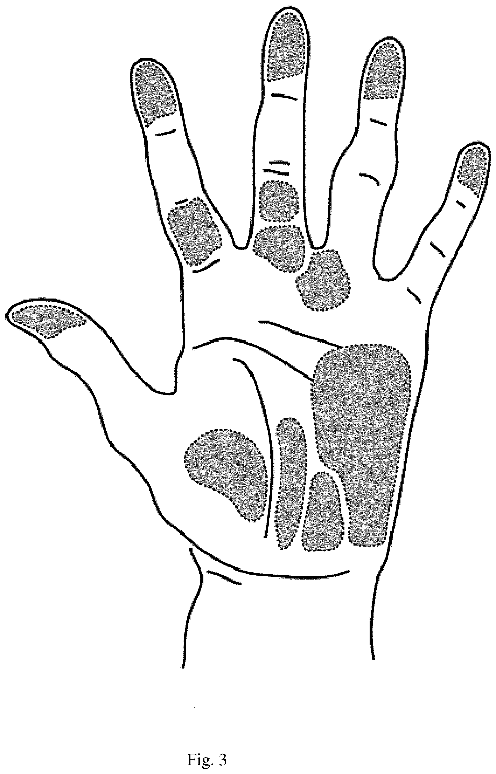

Areas of maximum accumulation of tactile mechanoreceptors called as Meissner bodies on the inner side of the palm are identified on Fig. 3.

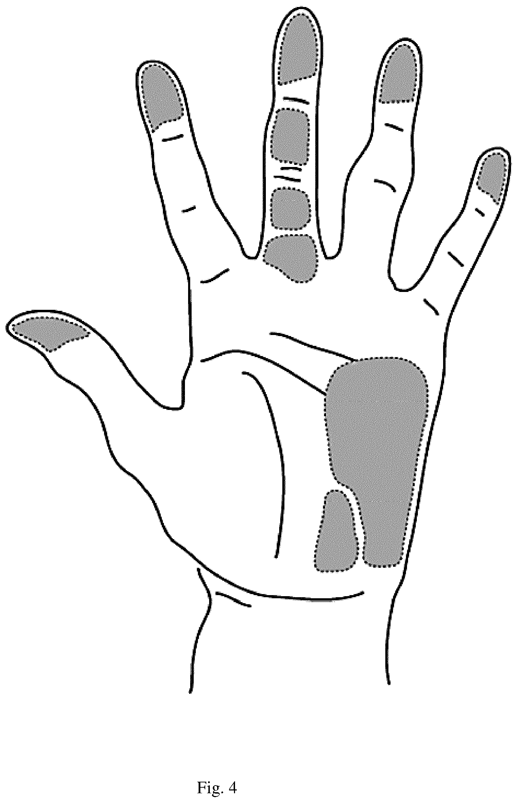

Areas of maximum accumulation of tactile mechanoreceptors called as Pacinian bodies on the inner side of the palm are identified on Fig. 4.

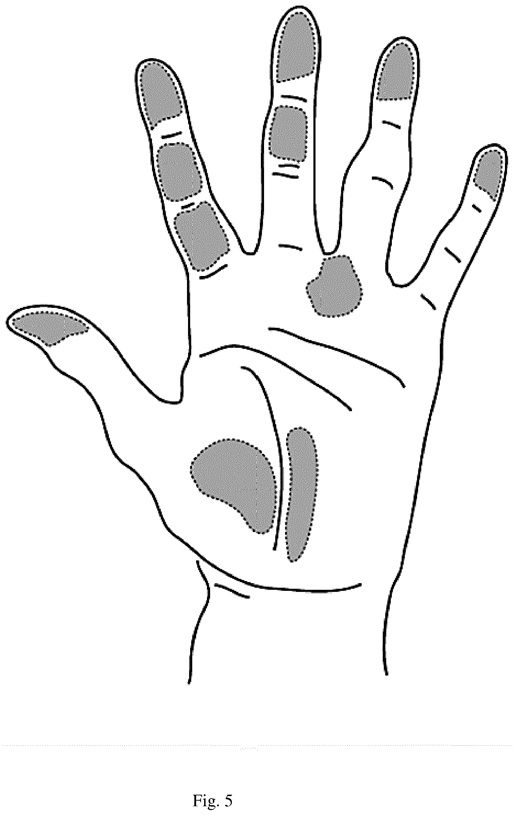

Areas of maximum accumulation of tactile mechanoreceptors called as Merkel nerve endings on the inner side of the palm are identified on Fig. 5.

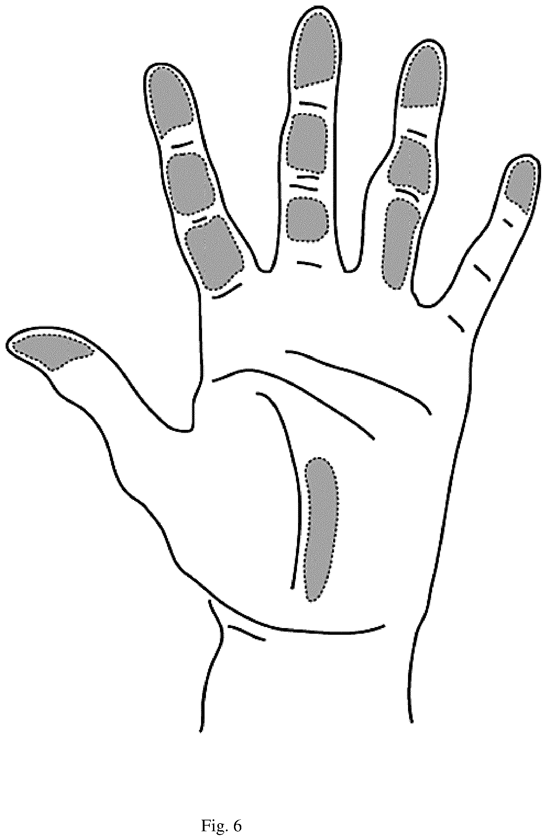

Areas of maximum accumulation of tactile mechanoreceptors called as Ruffini and Krause bodies on the inner side of the palm are identified on Fig. 6.

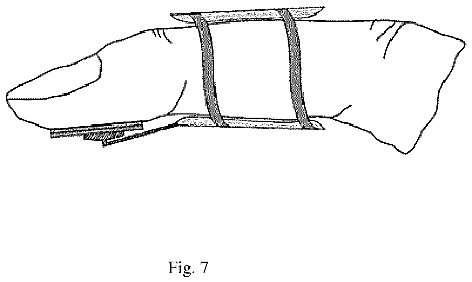

A variant of arrangement of actuation elements on the receptive field in the area of the tip of the index finger is shown on Fig. 7.

### Detailed disclosure of the present invention

The term "exteroceptive sensations" used in the present invention means sensations that are brought to the consciousness of a person by information from the external world.

Preferred exteroceptive sensations generated by the device according to the present invention are contact exteroceptive sensations, namely, tactile and temperature sensations.

In the device according to the present invention, a tactile method of receiving information is used to create contact exteroceptive sensations. Sensations are imitated through exposure to the receptive field by various external stimulants.

In one embodiment, a glove-shaped device is provided (see Fig. 1), where stimulants acting on the receptive field are located on the inner side of the phalanges of the fingers of the hand and on the inner side of the palm of the hand.

Actuation of stimulants is directed to tactile mechanoreceptors, which perceive phenomena such as touch, pressure, skin stretching and temperature changes. The affected elements in this receptive field are the free nerve endings with the lost myelin layer and neuroleum, the bodies of tactile mechanoreceptors of Meissner, Pacinian, Ruffini and Krause, and Merkel nerve endings.

A detailed description of the general functions and characteristics of mechanoreceptors can be found in the book "Neuroscience. 2nd edition ", released in 2001 by Sinauer Associates at Oxford University. The corresponding section is available here: https://www.ncbi.nlm.nih.gov/books/NBK11162/.

Description of mechanoreceptors of Meissner, Pacinian, Ruffini and Krause indicating their functions disclosed in the book D. J. Taylor, N. P. O. Green, G. W. Stout, R. Soper, "Biological Science 1&2", v. 2, Third ed., 1997, Cambridge University Press, Sections 16.5.1. "Mechanoreceptors" and 16.5.2. "Thermoreceptors". In the cited book, in particular, it is claimed that specialized receptors called Meissner bodies, which lie under the epidermis and consist of one convoluted nerve ending enclosed in a capsule filled with liquid, respond to touch. In the skin, joints, tendons, muscles and mesentery, there are Pacinian bodies consisting of the end of one neuron surrounded by connective tissue plates. These receptors respond to pressure. It is also indicated that there are two types of receptors in the dermis that are thought to be responsible for temperature sensitivity: Ruffini bodies that respond to heat and Krause cones that respond to cold.

Merkel bodies (or Merkel discs) that perceive tactile irritations, their location and functions are described in Halata Z., Baumann K. I., Grim M. Merkel Nerve Endings Functioning as Mechanoreceptors in Vertebrates // The Merkel Cell: Structure - Development - Function - Cancerogenesis / Baumann K. I., Halata Z., Moll I. (Eds.). - Berlin, Heidelberg: Springer Verlag, 2003.

In one embodiment of the device according to the present invention, areas of actuation in the receptive field are defined at the place of maximum concentration of the corresponding tactile mechanoreceptors (see Fig. 2.)

By acting in a receptive field on tactile mechanoreceptors by direct stimulation of sensory neurons, one can simulate different sensations.

Providing stimulation in the form of a vibrational actuation on the receptive field, for example, by producing an alternating electric current changing the shape of the piezoelectric element that comes in contact with the skin in the actuation area in the frequency range from 30 to 40 Hz, tactile mechanoreceptors called as Meissner bodies are activated. Their maximum congestions are marked on Fig. 3. This actuation simulates a weak direct touch due to the surface location of the bodies in the epidermis.

In course of actuation in the form of vibrational actuation in the frequency range from 70 to 800 Hz, the tactile mechanoreceptors called as Pacinian bodies are activated. The areas of their maximum congestion are marked on Fig. 4. This actuation simulates a strong direct touch.

In course of simultaneous actuation of Merkel nerve endings, which maximum congestion areas are marked on Fig. 5, the actuation is performed by displacing the skin surface in the stimulation zone in the range from 1 to 2 millimetres, while the contact of the skin surface in the area of contact with the stimulator is maintained. This actuation leads to a slight tension of the skin surface behind the perimeter of the exposure area, which, in combination with imitation of touch, simulates a sensation of touch movement on the surface of the actuation area.

At the same time, a wide range of frequency actuation on tactile mechanoreceptors, i.e. Pacinian bodies, allows to simulate the surface texture by short-term changing the frequency of exposure, thus simulating a smooth or rough surface.

The simultaneous and directed actuation of vibration stimulators and displacement stimulators along the receptive surface can simulate the touch direction of movement along the surface. In this case, the sharpness and degree of displacement, in combination with an increase or decrease in the speed of vibration actuation described above, simulates the sharpness of the movement start and the speed of movement over the surface.

By exerting a thermal actuation in the area of the receptive field to a depth of more than 0.17 mm, tactile mechanoreceptors, i.e. Ruffini bodies, are activated. The areas of their maximum congestion are marked on Fig. 6. These receptors are receptors of thermal sensitivity, which, in combination with the above actuation, mimics the sensation of an elevated temperature of the touched simulated surface. The thermal actuation can be performed for example by means of a thermal element that comes into contact with the skin in the receptive area. The thermal element, called the Peltier element, can be used. The element consists of two dissimilar conductors. When an electric current passes through the place of connection of two dissimilar conductors, release of energy in the place leads to heating.

By exerting a cooling actuation in the area of the receptive field to a depth of more than 0.3 mm, tactile mechanoreceptors, i.e. Krause bodies, are activated. The areas of their maximum congestion are marked on Fig. 6. These receptors are receptors of cold sensitivity, which, in combination with the above actuation, simulates the sensation of an reduced temperature of the touched simulated surface. The thermal actuation can be performed for example by means of a thermal element that comes into contact with the skin in the receptive area. The thermal element, called the Peltier element, can be used. The element consists of two dissimilar conductors. When an electric current passes through the place of connection of two dissimilar conductors, absorption of energy in the place leads to cooling.

In particular, in this described embodiment of the present invention using a Peltier element, it is possible to change the temperature of the exposure zone in the range from plus 40 to minus 5 degrees, which can be adjusted by changing the direction and strength of the electric current, while the contact to the skin surface in the exposure area is maintained.

By exerting mechanical pressure in the area of the receptive field, for example, by means of primitive mechanical elements and methods, tactile mechanoreceptors, i.e. Pacinian bodies, are activated. These receptors are also pressure-sensitive receptors, which activation simulates a feeling of pressure on the surface.

The simulation of the direction of movement along the surface, the formation of a touchable, embossed texture, the strength of touching it, as well as the surface temperature is achieved by combining the above simulated processes regulated and controlled by a single system.

Control of the stimulators located on the device according to the present invention is performed by software installed on a computer or smartphone through a control and communication unit connected to the specified stimulators, preferably via wireless connection by Wi-Fi or Bluetooth.

The device is powered by a power supply located on the specified device.

One embodiment of the device according to the present invention is depicted on Fig. 1.

For example, in order to simulate the slow light touching movement of an open palm along the vertical, smooth, cool surface from top to bottom using the glove-shaped device according to the present invention, it is necessary to simultaneously act on the receptive field defined in Fig. 2, by the following stimulants:
- vibration stimulators at a constant frequency in the range from 200 to 500 Hz,
- stimulators of displacement of the skin surface upward in the range from 0.3 to 0.5 mm,
- cooling stimulants in the temperature range from 12 to 18 degrees Celsius.

A variant of displacement of stimulants acting on the receptive field in the area of the index finger is shown on Fig. 7 for the device in the glove-shaped form of the present invention.

While the present inventions are described in detail above, one skilled in the art will recognize that modifications and equivalent substitutions can be made, and such modifications and substitutions are within the scope of the present inventions defined by the appended claims.

## Claims

1. A device for simulating and transmitting contact exteroceptive sensations comprising:
- a glove made of elastic material,
- stimulants of mechanical, dynamic and thermal effects fixed at the glove areas contacting with the fingers and palm of the hand at the areas of location of Meissner's, Pacinian, Ruffini's and Krause's mechanoreceptors, and Merkel nerve endings,
- control and communication unit connected to these stimulants and communicating with control software of a computer or a smartphone,
- power supply.

2. The device of claim 1, **characterized in that** the stimulants are located on the inner side and outer side of the palm of the hand and the phalanges of the fingers.

3. The device of claim 1, **characterized in that** the device additionally comprises a fixing member on the arm wrist.

4. A method of simulating and transmitting contact exteroceptive sensations by stimulating receptors using the device according to claim 1, in which:
- sensations of surface texture are imitated by vibrational stimulation of the tactile mechanic receptors of Meissner and Pacini,
- the sensation of directed touching movement along the surface is imitated by vibrational stimulation of the tactile mechanic receptors of Meissner and Pacini in synchronization with stimulation of Merkel nerve endings by displacement of the skin,
- the sensation of surface temperature is imitated by thermal stimulation of the tactile mechanoreceptors of Ruffini and Krause,
- the sensation of touching the surface is imitated by pressure on the tactile Pacini mechanoreceptors.
